# FASCICULE DE BREVET EUROPEEN

(11) **EP 3 614 991 B1**
(45) Date de publication et mention de la délivrance du brevet: **12.01.2022**
(21) Numéro de dépôt: 18720583.6
(22) Date de dépôt: 25.04.2018
(51) Int. Cl.: A61K 8/25, A61K 8/86, A61K 8/85, A61K 8/37, A61K 8/02, A61Q 3/02, A61K 8/04, A61K 8/60, A61K 8/81, A61K 8/84

(54) **RÉDUCTION DE LA TURBIDITÉ DE VERNIS À ONGLES**
VERRINGERUNG DER TRÜBUNG VON NAGELLACK
REDUCING THE TURBIDITY OF NAIL VARNISH

(30) Priorité: 25.04.2017 LU 100176
(43) Date de publication de la demande: 04.03.2020
(73) Titulaire: International Lacquers S.A., 3225 Bettembourg (LU)
(72) Inventeur: CISNEROS, Robin, 57070 Saint Julien lès Metz (FR); DYLEWICZ, Carole, 57180 Terville (FR); EDDOUMY, Fatima, 57840 Ottange (FR); DEROSIER, Frédéric, 57680 Corny sur Moselle (FR)
(74) Mandataire: Office Freylinger
(86) Numéro de dépôt international: PCT/EP2018/060605
(87) Numéro de publication internationale: WO 2018/197566

(56) Documents cités:
- US-A- 5 725 866
- US-A1- 2001 007 676
- US-A1- 2016 122 486
- US-B1- 6 352 687
- DATABASE GNPD [Online] MINTEL; 1 mai 2016 (2016-05-01), "Top Coat", XP002775074, Database accession no. 3952191

## Description

### Domaine technique

La présente invention concerne l'amélioration de la transparence ou la réduction de la turbidité de (bases de) vernis à ongles contenant des constituants particulaires en suspension par exemple, des nacres, des glitters (paillettes), des particules métalliques, etc.

### Etat de la technique

Les vernis à ongles sont généralement utilisés pour maquiller les ongles ainsi que les faux ongles. Les vernis à ongles sont habituellement composés d'un agent filmogène, d'un solvant, et éventuellement d'un ou plusieurs plastifiants, d'une ou plusieurs résines, d'un ou plusieurs agents de rhéologie et un ou plusieurs colorants.

Pour répondre aux exigences des utilisateurs, les vernis à ongles doivent satisfaire plusieurs critères :
- une bonne application, lorsque le vernis à ongles est appliqué sur l'ongle, il doit former une surface lisse, sans stries avec une épaisseur constante afin d'obtenir un film et une coloration homogènes,
- un étalement facile sur toute la surface de l'ongle pour rendre son utilisation pratique,
- un temps de séchage et de durcissement sur l'ongle court,
- une bonne tenue du vernis à ongles sur l'ongle, il est en effet souhaité que le vernis s'écaille le moins possible afin de conserver un aspect esthétique,
- de bonnes propriétés de conservation et de stabilité, en particulier une absence totale de sédimentation dans le temps, et
- un degré de turbidité le plus bas possible en particulier dans le cas où le vernis contient des nacres, des glitters (paillettes), des particules métalliques, etc.

L'introduction de constituants particulaires dans les vernis à ongles pose d'une manière générale le problème de la stabilité dans le temps. En effet, il est important pour l'utilisateur de pouvoir juger l'apparence d'un vernis sur les ongles à partir de son aspect dans le flacon sans devoir le secouer pendant une durée qui sera de toute façon jugée trop longue. Or, il est connu que toute dispersion est thermodynamiquement instable et de par ce principe la sédimentation serait donc la conséquence inévitable, surtout pour des grandes particules comme les nacres, les glitters et les particules métalliques. Cependant cette instabilité peut être contrée en contrôlant la cinétique de sédimentation. Classiquement les vernis à ongles comprenant des constituants particulaires contiennent donc en général des agents d'ajustement de la rhéologie ou de la thixotropie.

Cependant, il y a d'autres exigences notamment dans le cas de vernis à ongles comprenant des constituants particulaires, mais qui sont censé être transparents (donc non fortement pigmentés). Les ingrédients utiles pour contrôler la cinétique de sédimentation doivent dans ce cas en plus ne pas rendre la composition trouble.

Ceci est d'autant plus important que l'incorporation-même des constituants particulaires dans une composition peut engendrer une valeur de trouble plus élevée, réduisant ainsi (encore) la transparence apparente de la composition.

L'utilisation de silice pyrogénée a depuis longtemps été proposée en remplacement des traditionnelles bentones comme agent rhéologiques capables de fournir des vernis à ongles translucides (US 6 352 687 B1, US 2001/007676 A1, US 2016/122486 A1). Néanmoins, la transparence des systèmes décrits dans la littérature n'est pas toujours très bonne, et la plupart des systèmes n'utilisant pas de solvants aromatiques sont plutôt translucides que transparents.

Un grand nombre de solutions ont été proposées, cependant au vu des résultats pas toujours satisfaisants, notamment l'utilisation de solvants aromatiques de type toluène ou xylène reconnus comme étant CMR (Cancérogène, Mutagène et Reprotoxique), il reste toujours un besoin de trouver d'autres solutions pour réduire la turbidité (ou améliorer la transparence) de compositions de (bases de) vernis à ongles, de préférence bien sûr sans en altérer (significativement) leurs autres propriétés importantes pour l'utilisateur, notamment la tenue, le temps de séchage, le durcissement, etc.

### Objet de l'invention

Un objet de la présente invention est par conséquent de trouver un moyen de stabiliser des compositions de vernis à ongles contenant des constituants particulaires tout en réduisant leur turbidité de manière à pouvoir proposer des compositions dont la fraction hors particules présente une bonne transparence.

### Description générale de l'invention

Afin de résoudre le problème mentionné ci-dessus, la présente invention propose l'utilisation d'une combinaison d'un ou de plusieurs agents de réduction de la turbidité et de silice pyrogénée hydrophile ou hydrophobe, de préférence hydrophobe pour augmenter la transparence optique et/ou la stabilité dans le temps, d'une composition (anhydre) de base de vernis à ongles ou d'une composition (anhydre) de vernis à ongles comprenant des constituants particulaires comme les nacres, les glitters (paillettes) et/ou les particules métalliques, la composition de base de vernis à ongles, respectivement la composition de vernis à ongles étant exemptes de toluène et de xylène, et le ou les agents de réduction de la turbidité étant choisis parmi (a) les composés non-polymères benzoates de saccharose ayant un poids moléculaire supérieur à 700 Da et comprenant n groupements aryles ou hétéroaryles, n étant supérieur ou égal à 4 et de préférence compris entre 5 et 20 ; et parmi (b) les composés oligomères ou polymères époxytosylamides, copolymères styrène-acryliques, copolymères phtaliques, ou leurs combinaisons, ayant un poids moléculaire supérieur à 1000 Da et comprenant m groupements aryles ou hétéroaryles, m étant supérieur ou égal à 10 et de préférence compris entre 15 et 1000.

Les inventeurs ont trouvé qu'en combinant un tel agent avec de la silice pyrogénée hydrophile ou hydrophobe, de préférence hydrophobe, il devient possible d'abaisser significativement la turbidité de compositions contenant des constituants particulaires comme les nacres, les glitters (paillettes) et/ou les particules métalliques. Ceci est d'autant plus intéressant que cette combinaison permet en plus de régler ou contrôler la thixotropie à des valeurs plus élevées, avantageuses pour la stabilité des compositions dans le flacon à long terme.

Dans le contexte de la présente invention, un groupement aryle peut être tout noyau aromatique ou hétéroaromatique (donc comprenant des liaisons π) simple ou condensé, substitué ou non. Notamment les groupements aryles et hétéroaryles peuvent être choisis indépendamment parmi les groupes phényle, naphtyle, pyridyle, thiényle, furyle, pyridazinyle, pirimidinyle, pyrazinyle, thiazolyle, imidazolyle ou oxazolyle, substitués ou non par un ou plusieurs groupes par exemple choisis parmi un groupe -OH, -COOH, alkyle en C₁ à C₄, un atome d'halogène, etc. Dans le cas des oligomères et polymères, les groupes aryles et hétéroaryles sont de préférence « latéraux », c'est-à-dire ceux qui sont fixés directement ou indirectement par l'entremise d'un groupement intermédiaire à la chaîne principale par un seul de leurs atomes. Ces groupes aryles et hétéroaryles « latéraux » ne sont donc pas être intégrés dans la chaîne principale par deux liaisons intra-chaînes. Les groupements intermédiaires peuvent être des groupes alkyles, oxyalkyles, alkyloxy, sulfonyles, sulfinyles, etc. D'une manière générale, les oligomères et polymères peuvent dans ce cas en plus contenir des groupements aryles ou hétéroaryles à l'intérieur de leur chaîne. Les polyuréthanes bien qu'ils puissent être utilisés dans le cadre de l'invention ne sont pas préférés.

Dans une variante particulièrement préférée, l'utilisation comprend des quantités d'agent(s) de réduction de la turbidité représente 1 à 12 % en poids, de préférence 2 à 10 % en poids, en particulier de 4 à 8 % en poids sec par rapport au poids total de la composition de base de vernis à ongles ou de la composition de vernis à ongles.

La composition de base de vernis à ongles ou la composition de vernis à ongles peut comprendre en outre au moins un solvant organique cosmétiquement acceptable et au moins un agent filmogène.

L'agent ou les agents de réduction de la turbidité sont donc choisis parmi les benzoates de saccharose, les époxytosylamides, les copolymères styrène-acryliques, les copolymères phtaliques ou leurs combinaisons.

L'utilisation selon l'invention peut en outre comprendre, dans la composition de base de vernis à ongles ou la composition de vernis à ongles, un ou plusieurs autres additifs choisis parmi les agents filmogènes, les résines, les plastifiants, les agents rhéologiques, les absorbants UV, les modificateurs de surface et les agents dits « traitants ».

Dans un deuxième aspect, l'invention propose un procédé de préparation d'une composition de base de vernis à ongles ou d'une composition de vernis à ongles, comprenant le mélange d'un solvant organique, d'un ou de plusieurs agents de réduction de la turbidité, de silice pyrogénée hydrophile ou hydrophobe, de préférence hydrophobe, et de constituants particulaires, notamment de nacres, de glitters et/ou de particules métalliques, la composition de base de vernis à ongles, respectivement la composition de vernis à ongles étant exemptes de toluène et de xylène, et le ou les agents de réduction de la turbidité étant choisis parmi (a) les composés non-polymères benzoates de saccharose ayant un poids moléculaire supérieur à 700 Da et comprenant *n* groupements aryles ou hétéroaryles, n étant supérieur ou égal à 4 et de préférence compris entre 5 et 20 ; et parmi (b) les composés oligomères ou polymères époxytosylamides, copolymères styrène-acryliques, copolymères phtaliques, ou leurs combinaisons, ayant un poids moléculaire supérieur à 1000 Da et comprenant *m* groupements aryles ou hétéroaryles, m étant supérieur ou égal à 10 et de préférence compris entre 15 et 1000.

Dans un tel procédé, la quantité d'agent(s) de réduction de la turbidité représente avantageusement 1 à 12 % en poids, de préférence 2 à 10 % en poids, en particulier de 4 à 8 % en poids sec par rapport au poids total de la composition de base de vernis à ongles ou de la composition de vernis à ongles.

Le procédé comprend en outre de préférence l'addition d'au moins un agent filmogène dans la composition de base de vernis à ongles ou la composition de vernis à ongles.

Les définitions et les préférences concernant les agents de réduction de la turbidité pour le procédé sont celles indiquées précédemment.

Finalement, le procédé selon l'invention comprend en outre dans certaines variantes, l'incorporation dans le mélange d'un ou de plusieurs autres additifs choisis parmi les agents filmogènes, les résines, les plastifiants, les agents rhéologiques, les absorbants UV, les modificateurs de surface et les agents dits « traitants ».

Le ou les agent(s) filmogène(s) compris de préférence dans la composition de base de vernis à ongles ou composition de vernis à ongles peu(ven)t être choisi(s) parmi le groupe constitué par la cellulose, un dérivé de cellulose, une résine vinylique, une résine acrylique et n'importe quelle combinaison de ceux-ci. Les dérivés de cellulose sont par exemple la nitrocellulose, l'acétate butyrate cellulose, l'éthylcellulose et l'hydroxypropylcellulose. Préférentiellement, on choisira la nitrocellulose comme agent filmogène.

Un agent filmogène seul ou une combinaison d'agents filmogènes peut être utilisé(e) dans la composition de base de vernis à ongles ou composition de vernis à ongles dans les proportions allant de 5 % à 20 % en poids sec et de façon préférée de 12 % à 16 % en poids sec par rapport au poids total de la composition.

Selon un mode de réalisation de l'invention, la composition de base de vernis à ongles ou composition de vernis à ongles comprend un solvant organique, en plus du solvant éventuellement utilisé pour solubiliser la résine de copolymères d'acide adipique, d'acide fumarique, de néopentylglycol et de cyclohexanediméthanol, en particulier la résine de Polyester-26. Ce solvant organique peut être choisi parmi le groupe constitué par l'acétate de méthyle, l'acétate d'éthyle, l'acétate de butyle, l'acétate d'isobutyle, l'acétate de propyle, une cétone, un ester, un alcool, tel que l'éthanol dénaturé (avec la méthyléthylcétone ou le diéthyléther), l'isopropanol, le butanol, le diacétone alcool, les hydrocarbures linéaires ou cycliques, comme par exemple l'hexane, le cyclohexane, l'heptane et n'importe quelle combinaison de ceux-ci.

Le solvant organique pourra être choisi en fonction de ses propriétés physico-chimiques et en considérations des autres composants présents dans la composition de base de vernis à ongles ou composition de vernis à ongles. En outre de façon préférée, la quantité de solvant organique présente dans la composition de base de vernis à ongles ou composition de vernis à ongles est comprise entre 60% et 80% en poids par rapport au poids total de la composition.

Avantageusement, le solvant organique est l'acétate d'éthyle et/ou l'acétate de butyle et/ou de l'alcool.

En plus, de la résine de copolymères d'acide adipique, d'acide fumarique, de néopentylglycol et de cyclohexanediméthanol, en particulier la résine de Polyester-26, des résines supplémentaires (différentes) peuvent également être ajoutées dans la composition. En particulier, ces résines supplémentaires permettent d'augmenter le collant et l'adhérence du film sur l'ongle.

Les résines pouvant être utilisées sont notamment les suivantes :
- sucrose acétate isobutyrate,
- résine polyvinylbutyral.

En plus de leur action collante, la plupart de ces résines additionnelles ont également une action plastifiante. Les résines additionnelles peuvent être utilisées dans la formule, seules ou sous la forme de mélanges dans des quantités allant de 3 à 20 % en poids sec, de préférence de 5 à 15% en poids sec, en particulier de 7 à 12 % en poids sec par rapport au poids total de la composition.

Selon un mode de réalisation, la composition de base de vernis à ongles ou composition de vernis à ongles peut également comprendre des plastifiants. Les plastifiants sont des composés ayant une température d'ébullition élevée qui ne s'évaporent pas lors du séchage du vernis à ongles. Ils sont utilisés pour moduler la dureté du film formé afin d'obtenir les caractéristiques physico-chimiques du film souhaitées et représentent en général de 1 à 12 % en poids sec, de préférence 5 à 10 % en poids sec, en particulier de 6 à 8 % en poids sec par rapport au poids total de la composition.

Des exemples de plastifiants utilisables dans l'invention sont l'acétyl tributyl citrate, l'acétyl triéthyl citrate, le tributyl citrate, le triéthylcitrate, le dibutyl phtalate, le triphénylphosphate, la triacétine, le triméthyl pentanyl diisobutyrate, la triéthylhexanoïne, le sucrose benzoate, le dibutyl adipate, le diéthyl phthalate, le diisobutyl adipate, le diisopropyl adipate, le dipropylène glycol dibenzoate, etc.

Les compositions pour vernis à ongles utilisables dans le contexte de l'invention comprennent un ou plusieurs agents choisis parmi les nacres, les glitters (paillettes) et/ou les particules métalliques. Ces agents fournissent l'aspect esthétique recherché par les utilisateurs de vernis à ongles. Ces agents peuvent produire des effets très différents tels que « irisé », « métallisé », « nacré », « miroir », « pailleté », « craquelé », « mat », « néon », « fluorescent », « holographique », etc.

Les nacres dans le contexte de l'invention sont des particules minérales de différentes natures recouvertes d'une ou plusieurs couches d'oxydes (oxydes de titane ou oxydes de fer) ou encore de pigments. Les nacres peuvent se présenter sous la forme d'une poudre ou de plaque de tailles variées. Parmi les nacres utilisables dans l'invention, on peut citer :
- les nacres préparées à partir de mica naturel et ayant subi des opérations de coating successives,
- les nacres préparées à partir de mica synthétique appelé également « synthetic fluorphlogopite »,
- les nacres préparées à partir de calcium sodium borosilicate,
- les nacres préparées à partir d'oxyde de silice.

Les glitters (paillettes) dans le contexte de l'invention peuvent être préparés à partir de plusieurs types de matériaux comme les films polyesters comportant en surface une couche métallique, le polyéthylène téréphtalate, le polybutylène téréphtalate, les copolymères d'acrylates, les copolymères d'acrylates ou encore l'aluminium.

La composition de base de vernis à ongles ou composition de vernis à ongles peut en outre comprendre divers additifs choisis parmi les agents filmogènes, les résines, les agents rhéologiques, les modificateurs de surface et les agents dits « traitants ».

Si nécessaire ou souhaité, les compositions peuvent également comporter des absorbeurs UV. Parmi ces absorbants UV, on citera : les résines de copolymère(s) d'acide adipique, d'acide fumarique, de néopentylglycol et de cyclohexanediméthanol, notamment la résine de Polyester-26 (désignation INCI), la benzophénone-1 (CAS 131-56-6), la benzophénone-3 (CAS 131-57-7), le benzyl salicylate (CAS 118-58-1), l'étocrylène (CAS 5232-99-5), le drométrizole (CAS 2440-22-4), le butyl méthoxydibenzoylméthane (CAS 70356-09-1), etc.

Les absorbants UV seront utilisés seuls ou sous la forme de combinaison dont le pourcentage total dans la formule peut aller de 0 à 1,5 % en poids sec par rapport au poids total de la composition.

Parmi les additifs utilisables selon l'invention, on citera également les modificateurs de surface comme les cyclométhicones ou cyclopentasiloxane, les diméthicones et le triméthylsiloxysilicate, Butyl Acrylate/hydroxypropyldimethicone Acrylate Copolymer. Ces additifs de surface sont généralement utilisés dans des faibles quantités dans la formulation allant de 0,1 à 5 % en poids sec par rapport au poids total de la composition.

On citera également les additifs dits « traitants » de l'ongle utilisables dans la composition selon l'invention. Les additifs traitants peuvent représenter de 0,01 % à 5 % en poids sec, de préférence de 0,05 % à 2 % en poids sec par rapport au poids total de la composition. Parmi ces additifs « traitants », on citera :
- le formaldéhyde utilisé comme durcisseur de l'ongle,
- la vitamine E acétate (tocophéryl acétate),
- la vitamine A palmitate (rétinyl palmitate),
- les dérivés organiques ou inorganiques de calcium tels que le chlorure de calcium, le pantothénate de calcium,
- le diméthyloxobenzodioxasilane,
- la myrrhe,
- les acides aminés soufrés comme la cystéine, ses sels et leurs dérivés, la cystine, le glutathion. Les acides aminés soufrés sont en effet capables de créer des ponts de réticulation entre la kératine et les groupes SH libres de ces dérivés soufrés,
- la kératine hydrolysée d'origine végétale,
- les alpha-hydroxyacides comme l'acide citrique, l'acide ascorbique,
- la biotine,
- l'urée et la diméthylurée.

Tous les modes de réalisation cités précédemment peuvent être combinés sous réserve de leur faisabilité technique.

### Exemples

Les compositions suivantes Ex. 1 à Ex. 3 ont été préparées suivant le procédé décrit précédemment en mélangeant les ingrédients mentionnés dans le tableau suivant :

| | Ex.1 | Ex. 2 | Ex. 3 |
|---|---|---|---|
| Acétate d'éthyle | 49,34 | 46,55 | 49,34 |
| Acétate de butyle | 12,59 | 13,35 | 12,59 |
| Alcool isopropylique | 4,07 | 4,58 | 4,07 |
| Nitrocellulose | 9,49 | 10,68 | 9,49 |
| Phtallic anhydride / trimellitic anhydride / glycols copolymer | 6,75 | 4,30 | 6,75 |
| Perbenzoate de saccharose | 2,50 | 3,00 | 2,50 |
| Epoxy tosylamide | 11,25 | 12,65 | 11,25 |
| ATBC | 2,32 | 2,90 | 2,32 |
| Butyl Acrylate/hydroxypropyldimethicone Acrylate Copolymer | 0,10 | | 0,10 |
| Silice pyrogénée | (hydrophile) 1,6 | (hydrophobe) 2 | (hydrophobe) 1,6 |
| | 100,00 | 100,00 | 100,00 |
| Turbidité (abs/cm)* | 0,075 | 0,072 | 0,068 |

| | | | |
|---|---|---|---|
| * La turbidité a été mesurée au moyen d'un spectrophotomètre Jenway, Genova Plus Life Science Spectrophotometer. | | | |

On peut constater que les résultats de turbidités sont extrêmement bas, c'est-à-dire les compositions des exemples sont très transparentes. Pour comparaison, une base transparente sur le marché actuellement possède une valeur turbidité de 0.512 abs/cm.

## Revendications

1. Utilisation d'une combinaison d'un ou de plusieurs agents de réduction de la turbidité et de la silice pyrogénée hydrophile ou hydrophobe, de préférence hydrophobe, pour augmenter la transparence optique et/ou la stabilité dans le temps d'une composition de base de vernis à ongles ou d'une composition de vernis à ongles comprenant des constituants particulaires, notamment des nacres, des glitters et/ou des particules métalliques, la composition de base de vernis à ongles, respectivement la composition de vernis à ongles étant exemptes de toluène et de xylène, et le ou les agents de réduction de la turbidité étant choisis parmi (a) les composés non-polymères benzoates de saccharose ayant un poids moléculaire supérieur à 700 Da et comprenant *n* groupements aryles ou hétéroaryles, *n* étant supérieur ou égal à 4 et de préférence compris entre 5 et 20 ; et parmi (b) les composés oligomères ou polymères époxytosylamides, copolymères styrène-acryliques, copolymères phtaliques, ou leurs combinaisons, ayant un poids moléculaire supérieur à 1000 Da et comprenant *m* groupements aryles ou hétéroaryles, *m* étant supérieur ou égal à 10 et de préférence compris entre 15 et 1000.

2. Utilisation selon la revendication 1, dans laquelle les agents de réduction de la turbidité comprennent au moins un composé non-polymère (a) et au moins un composé oligomère ou polymère (b).

3. Utilisation selon la revendication 1 ou 2, dans laquelle la quantité d'agent(s) de réduction de la turbidité représente 1 à 12 % en poids, de préférence 2 à 10 % en poids, en particulier de 4 à 8 % en poids sec par rapport au poids total de la composition de base de vernis à ongles ou de la composition de vernis à ongles.

4. Utilisation selon l'une des revendications 1 à 3, dans laquelle la composition de base de vernis à ongles ou la composition de vernis à ongles comprend en outre au moins un solvant organique cosmétiquement acceptable et au moins un agent filmogène.

5. Utilisation selon l'une quelconque des revendications 1 à 4, dans laquelle la composition de base de vernis à ongles ou la composition de vernis à ongles comprend en outre un ou plusieurs autres additifs choisis parmi les agents filmogènes, les résines, les plastifiants, les agents rhéologiques, les absorbants UV, les modificateurs de surface et les agents dits « traitants ».

6. Procédé de préparation d'une composition de base de vernis à ongles ou d'une composition de vernis à ongles, comprenant le mélange d'un solvant organique, d'un ou de plusieurs agents de réduction de la turbidité, de silice pyrogénée hydrophile ou hydrophobe, de préférence hydrophobe, et de constituants particulaires, notamment de nacres, de glitters et/ou de particules métalliques, la composition de base de vernis à ongles, respectivement la composition de vernis à ongles étant exemptes de toluène et de xylène, et le ou les agents de réduction de la turbidité étant choisis parmi (a) les composés non-polymères benzoates de saccharose ayant un poids moléculaire supérieur à 700 Da et comprenant *n* groupements aryles ou hétéroaryles, *n* étant supérieur ou égal à 4 et de préférence compris entre 5 et 20 ; et parmi (b) les composés oligomères ou polymères époxytosylamides, copolymères styrène-acryliques, copolymères phtaliques, ou leurs combinaisons, ayant un poids moléculaire supérieur à 1000 Da et comprenant *m* groupements aryles ou hétéroaryles, *m* étant supérieur ou égal à 10 et de préférence compris entre 15 et 1000, le procédé comprenant de préférence en outre l'incorporation dans le mélange d'un ou de plusieurs autres additifs choisis parmi les agents filmogènes, les résines, les plastifiants, les agents rhéologiques, les absorbants UV, les modificateurs de surface et les agents dits « traitants ».

7. Procédé selon la revendication 6, dans lequel les agents de réduction de la turbidité comprenant au moins un composé non-polymère (a) et au moins un composé oligomère ou polymère (b).

8. Procédé selon la revendication 6 ou 7, dans lequel la quantité d'agent(s) de réduction de la turbidité représente 1 à 12 % en poids, de préférence 2 à 10 % en poids, en particulier de 4 à 8 % en poids sec par rapport au poids total de la composition de base de vernis à ongles ou de la composition de vernis à ongles.

9. Procédé selon l'une des revendications 6 à 8, dans lequel la composition de base de vernis à ongles ou la composition de vernis à ongles comprend en outre au moins un agent filmogène.

## Patentansprüche

1. Verwendung einer Kombination aus mindestens einem Mittel zur Reduzierung der Trübheit und hydrophiler oder hydrophober, vorzugsweise hydrophober, pyrogener Kieselsäure zur Erhöhung der optischen Transparenz und/oder der zeitlichen Stabilität einer Nagellack-Grundzusammensetzung oder einer Nagellackzusammmensetzung, umfassend teilchenförmige Komponenten, insbesondere Perlmutt, Glitter und/oder Metallpartikel, wobei die Grundzusammensetzung bzw. die Nagellackzusammensetzung frei von Toluol und Xylol ist und das mindestens eine Mittel zur Reduzierung der Trübheit aus folgender Gruppe gewählt ist : (a) nicht polymere Saccharosebenzoatverbindungen mit einem Molekulargewicht größer 700 Da und umfassend *n* Aryl- oder Heteroarylgruppen, wobei *n* größer oder gleich 4 ist und vorzugsweise zwischen 5 und 20 liegt, und (b) oligo- oder polymere Epoxidtosylamidverbindungen, Styrol-Acryl-Copolymere, Phthalsäurecopolymere oder Kombinationen davon mit einem Molekulargewicht größer 1000 Da, und umfassend *m* Aryl- oder Heteroarylgruppen, wobei *m* größer oder gleich 10 ist und vorzugsweise zwischen 15 und 1000 liegt.

2. Verwendung nach Anspruch 1, wobei die Mittel zur Reduzierung der Trübheit mindestens eine nicht polymere Verbindung (a) und mindestens eine oligo- oder polymere Verbindung (b) umfasst.

3. Verwendung nach Anspruch 1 oder 2, wobei die Menge des mindestens einen Mittels zur Reduzierung der Trübheit, bezogen auf das Gesamttrockengewicht der Grundzusammensetzung bzw. der Nagellackzusammensetzung, 1 - 12 Gew.-%, vorzugsweise aber 2 - 10 Gew.-%, insbesondere 4 - 8 Gew.-% beträgt.

4. Verwendung nach einem der Ansprüche 1 bis 3, wobei die Grundzusammensetzung bzw. die Nagellackzusammensetzung ferner mindestens ein kosmetisch verträgliches organisches Lösemittel und mindestens einen Filmbildner umfasst.

5. Verwendung nach einem der Ansprüche 1 bis 4, wobei die Grundzusammensetzung bzw. die Nagellackzusammensetzung ferner mindestens einen anderen aus folgender Gruppe gewählten Zusatzstoff umfasst: Filmbildner, Harze, Weichmacher, Fließmittel, UV-Absorptionsmittel, Oberflächenmodifikatoren und sog. "Behandlungsmittel".

6. Verfahren zur Erzeugung einer Grundzusammensetzung für Nagellack bzw. einer Nagellackzusammensetzung, umfassend Vermischen eines organischen Lösemittels, mindestens eines Mittels zur Reduzierung der Trübheit, hydrophiler bzw. hydrophober, vorzugsweise hydrophober, pyrogener Kieselsäure und teilchenförmiger Komponenten, insbesondere Permutt, Glitter und/oder Metallpartikel, wobei die Grundzusammensetzung bzw. die Nagellackzusammensetzung frei von Toluol und Xylol ist und das mindestens eine Mittel zur Reduzierung der Trübheit aus folgender Gruppe gewählt ist : (a) nicht polymere Saccharosebenzoatverbindungen mit einem Molekulargewicht größer 700 Da und umfassend *n* Aryl- oder Heteroarylgruppen, wobei n größer oder gleich 4 ist und vorzugsweise zwischen 5 und 20 liegt, und (b) oligo- oder polymere Epoxidtosylamidverbindungen, Styrol-Acryl-Copolymere, Phthalsäurecopolymere oder Kombinationen davon mit einem Molekulargewicht größer 1000 Da, und umfassend *m* Aryl- oder Heteroarylgruppen, wobei *m* größer oder gleich 10 ist und vorzugsweise zwischen 15 und 1000 liegt, wobei das Verfahren vorzugsweise ferner umfasst: Aufnehmen mindestens eines aus folgender Gruppe gewählten weiteren Zusatzstoffs: Filmbildner, Harze, Weichmacher, Fließmittel, UV-Absorptionsmittel, Oberflächenmodifikatoren und sog. "Behandlungsmittel".

7. Verfahren nach Anspruch 6, wobei die Mittel zur Reduzierung der Trübheit mindestens eine nicht polymere Verbindung (a) und mindestens eine oligo- oder polymere Verbindung (b) umfasst.

8. Verfahren nach Anspruch 6 oder 7, wobei die Menge des mindestens einen Mittels zur Reduzierung der Trübheit, bezogen auf das Gesamttrockengewicht der Grundzusammensetzung bzw. der Nagellackzusammensetzung, 1 - 12 Gew.-%, vorzugsweise aber 2 - 10 Gew.-%, insbesondere 4 - 8 Gew.-% beträgt.

9. Verfahren nach einem der Ansprüche 6 bis 8, wobei die Grundzusammensetzung bzw. die Nagellackzusammensetzung ferner mindestens einen Filmbildner umfasst.

## Claims

1. Use of a combination of one or more turbidity reducing agents and hydrophilic or hydrophobic fumed silica, preferably hydrophobic, in order to increase the optical transparency and/or the stability over time of a basic nail varnish composition or of a nail varnish composition comprising particulate constituents, in particular mother-of-pearls, glitters and/or metallic particles, the basic nail varnish composition, respectively the nail varnish composition being free from toluene and xylene, and the turbidity reducing agent(s) being selected from (a) non-polymer sucrose benzoate compounds having a molecular weight greater than 700 Da and comprising *n* aryl or heteroaryl groups, *n* being greater than or equal to 4 and preferably comprised between 5 and 20; and from (b) oligomer or polymer epoxytosylamide compounds, styrene-acrylic copolymers, phthalic copolymers, or their combinations, having a molecular weight greater than 1000 Da and comprising *m* aryl or heteroaryl groups, *m* being greater than or equal to 10 and preferably comprised between 15 and 1000.

2. The use according to claim 1, wherein the turbidity reducing agents comprise at least one non-polymer compound (a) and at least one oligomer or polymer compound (b).

3. The use according to claim 1 or 2, wherein the amount of turbidity reducing agent(s) represents 1 to 12% by weight, preferably 2 to 10% by weight, particularly 4 to 8% by dry weight based on the total weight of the basic nail varnish composition or of the nail varnish composition.

4. The use according to one of claims 1 to 3, wherein the basic nail varnish composition or the nail varnish composition further comprises at least one cosmetically acceptable organic solvent and at least one film-forming agent.

5. The use according to any one of claims 1 to 4, wherein the basic nail varnish composition or the nail varnish composition further comprises one or more other additives selected from film-forming agents, resins, plasticizers, rheological agents, UV absorbents, surface modifiers and "treatment" agents.

6. A process for preparing a basic nail varnish composition or a nail varnish composition, comprising mixing an organic solvent, one or more turbidity reducing agents, hydrophilic or hydrophobic fumed silica, preferably hydrophobic, and particulate constituents, in particular mother-of-pearls, glitters and/or metallic particles, the basic nail varnish composition, respectively the nail varnish composition being free from toluene and xylene, and the turbidity reducing agent(s) being selected from (a) non-polymer sucrose benzoate compounds having a molecular weight greater than 700 Da and comprising *n* aryl or heteroaryl groups, *n* being greater than or equal to 4 and preferably comprised between 5 and 20; and from (b) oligomer or polymer epoxytosylamide compounds, styrene-acrylic copolymers, phthalic copolymers, or their combinations, having a molecular weight greater than 1000 Da and comprising *m* aryl or heteroaryl groups, *m* being greater than or equal to 10 and preferably comprised between 15 and 1000, the process preferably further comprising the incorporation into the mixture of one or more other additives selected from film-forming agents, resins, plasticizers, rheological agents, UV absorbers, surface modifiers and "treatment" agents.

7. The process according to claim 6, wherein the turbidity reducing agents comprising at least one non-polymer compound (a) and at least one oligomer or polymer compound (b).

8. The process according to claim 6 or 7, wherein the amount of turbidity reducing agent(s) represents 1 to 12% by weight, preferably 2 to 10% by weight, particularly 4 to 8% by dry weight based on the total weight of the basic nail varnish composition or of the nail varnish composition.

9. The process according to one of claims 6 to 8, wherein the basic nail varnish composition or the nail varnish composition further comprises at least one film-forming agent.
